# EUROPÄISCHE PATENTSCHRIFT

(11) **EP 2 091 449 B1**
(45) Veröffentlichungstag und Bekanntmachung des Hinweises auf die Patenterteilung: **20.07.2016**
(21) Anmeldenummer: 07847389.9
(22) Anmeldetag: 27.11.2007
(51) Int. Cl.: A61B 17/84, A61B 17/70

(54) **IMPLANTAT UND IMPLANTATSYSTEM**
IMPLANT AND IMPLANT SYSTEM
IMPLANT ET SYSTÈME D'IMPLANT

(30) Priorität: 08.12.2006 DE 102006059395
(43) Veröffentlichungstag der Anmeldung: 26.08.2009
(73) Patentinhaber: Aesculap AG, 78532 Tuttlingen (DE)
(72) Erfinder: BEGER, Jens, 78532 Tuttlingen (DE); KLINGSEIS, Susanne, 88400 Biberach (DE)
(74) Vertreter: Hoeger, Stellrecht & Partner Patentanwälte mbB
(86) Internationale Anmeldenummer: PCT/EP2007/062862
(87) Internationale Veröffentlichungsnummer: WO 2008/068162

(56) Entgegenhaltungen:
- WO-A-02/03882
- FR-A- 2 717 675
- FR-A- 2 816 197
- US-A1- 2005 033 434
- US-A1- 2005 288 672

## Beschreibung

Die vorliegende Erfindung betrifft ein Implantat zur dorsalen Stabilisierung einer menschlichen oder tierischen Wirbelsäule umfassend eine Befestigungsvorrichtung zum Anlegen und/oder Festlegen an Dornfortsätzen benachbarter Wirbel der Wirbelsäule, welches Implantat mindestens ein der Befestigungsvorrichtung zugeordnetes Abstandselement umfasst, welches derart ausgebildet ist, dass es infolge einer Änderung von Umgebungsbedingungen und/oder auf es einwirkender Kräfte seine äußere Gestalt und/oder seine elastischen Eigenschaften ändert.

Ferner betrifft die vorliegende Erfindung ein Implantatsystem zur dorsalen Stabilisierung einer menschlichen oder tierischen Wirbelsäule mit mindestens einem Implantat zur dorsalen Stabilisierung der Wirbelsäule umfassend eine Befestigungsvorrichtung zum Anlegen und/oder Festlegen an Dornfortsätzen benachbarter Wirbel der Wirbelsäule mit einem Instrumentarium zum Einsetzen des Implantats in einen menschlichen oder tierischen Körper.

Ein Implantat zur dorsalen Stabilisierung einer menschlichen oder tierischen Wirbelsäule umfassend eine Befestigungsvorrichtung zum Anlegen und/oder Festlegen an Dornfortsätzen benachbarter Wirbel der Wirbelsäule ist beispielsweise aus der US 6,695,842 bekannt. Es dient der statischen dorsalen Stabilisierung der Wirbelsäule. Zu diesem Zweck wird es an Dornfortsätzen benachbarter Wirbel der Wirbelsäule festgelegt und hält diese dann in einem vorgegebenen Abstand, insbesondere um eine zwischen den beiden Wirbeln angeordnete Bandscheibe zu entlasten. Derartige Implantate werden insbesondere nach Bandscheibenoperationen eingesetzt oder dienen als zusätzliche Stabilisierung nach dem Entfernen einer Bandscheibe und anschließendem Verblocken benachbarter Wirbel miteinander.

Das bekannte Implantat eignet sich jedoch nicht, eine operativ behandelte Bandscheibe wieder sukzessive an eine natürliche Belastung zu gewöhnen. Es kann entweder zwei Wirbel relativ zueinander stabilisieren oder nach Entfernen des Implantats die beiden Wirbel vollständig freigeben. Dies hat zur Folge, dass entweder eine vollständige Entlastung der Bandscheibe oder eine vollständige Belastung der Bandscheibe bei Vorhandensein beziehungsweise nach dem Entfernen des Implantats erreicht wird.

Zwischen Dornfortsätze benachbarter Wirbelkörper eine Wirbelsäule einsetzbare Implantate sind aus der FR 2 816 197 A1 und der FR 2 717 675 A1 bekannt. In der WO 02/03882 A2 ist ein Stöße abfederndes Zwischenwirbelimplantat beschrieben. Vorrichtungen zum Verhindern der Ausdehnung einer Wirbelsäule sind in der US 2005/0288672 A1 offenbart. In der US 2005/ 0033434 A1 ist ein Wirbelsäulenstabilisierungssystem beschrieben.

Es ist daher Aufgabe der vorliegenden Erfindung, ein Implantat und ein Implantatsystem der eingangs beschriebenen Art so zu verbessern, dass sie zur dynamischen dorsalen Stabilisierung der Wirbelsäule verwendet werden können.

Diese Aufgabe wird bei einem Implantat der eingangs beschriebenen Art erfindungsgemäß dadurch gelöst, dass das Abstandselement hydrierbar ist, dass das mindestens eine hydrierbare Abstandselement mindestens teilweise aus einem Hydrogel hergestellt ist, dass das mindestens eine Abstandselement anisotrop verformbar ist, so dass es seine Form und/oder ein umschlossenes Volumen infolge einer Änderung mindestens einer Umgebungsbedingung anisotrop ändert, und dass das mindestens eine anisotrop verformbare Abstandselement seine Form und/oder ein umschlossenes Volumen infolge einer Hydrierung anisotrop ändert.

Mit einem derartigen Implantat ist es möglich, eine Balance zwischen einer Beweglichkeit der miteinander verbundenen, ein Bewegungssegment definierenden Wirbel und einer Stabilisierung zu erreichen. Es ermöglicht insbesondere eine Einschränkung einer Bewegung benachbarter Wirbel, und zwar je nach Ausgestaltung in Extension und/oder Flexion. Die durch das Implantat vorgebbare Beweglichkeit kann insbesondere an Änderungen von Umgebungsbedingungen angepasst sein. Beispielsweise hierfür geeignete Änderungen von Umgebungsbedingungen sind Feuchtigkeitsänderungen, Druckänderungen, Temperaturänderungen, Änderungen von pH-Werten und weitere Änderungen biologischer, chemischer und/oder physikalischer Größen in einer Umgebung des Implantats. Bewegungseinschränkungen können erreicht werden durch Formänderungen und/oder Änderungen elastischer Eigenschaften des mindestens einen Abstandselements, beispielsweise seines Elastizitätsmoduls. Je elastischer das Abstandselement ist, umso größer eine Beweglichkeit der miteinander verbundenen Wirbel und umgekehrt. Ferner kann das Abstandselement auch Lasten aufnehmen, die infolge einer Bewegung des Körpers auf die Wirbel einwirken. Mit der Befestigungsvorrichtung lässt sich insgesamt eine Entlastung der Bandscheibe zwischen den Wirbeln erreichen. Durch die dynamische dorsale Stabilisierung wird eine Regenerierung der Bandscheibe gefördert. Das vorgeschlagene Implantat kann insbesondere an Dornfortsätzen zweier benachbarter Wirbel festgelegt werden, wobei eine Verspannung des Implantats an den Dornfortsätzen nach Implantation mittels des mindestens einen Abstandselements erreicht werden kann. Ferner hat das Implantat den Vorteil, dass es auf physiologische Veränderungen reagieren kann, beispielsweise kann das Abstandselement aufgrund unterschiedlich einwirkender Kräfte beim Liegen und beim Gehen jeweils eine unterschiedliche Form und/oder unterschiedliche elastische Eigenschaften aufweisen, die entweder zu einer stärkeren Stabilisierung oder zu einer größeren Beweglichkeit der miteinander verbundenen Wirbel führen. Eine Beweglichkeit der Dornfortsätze relativ zueinander wird verbessert, wenn eine Elastizität des Abstandselements vergrößert wird. Die Stabilität der Verbindung zwischen den benachbarten Wirbeln wird dagegen verbessert, wenn die Elastizität des Abstandselements verringert wird. Erfindungsgemäß ist das Abstandselement hydrierbar. Ein derartiges Abstandselement weist besonders günstige Eigenschaften auf, um eine Adaption des Implantats an physiologische Änderungen im Bewegungssegment zu gestatten. So ist es bekannt, dass die Bandscheibenhöhe im Laufe eines Tages unter Belastung durch Dehydrierung abnimmt. Hier kann beispielsweise durch entsprechende Auswahl und Formgebung eines oder mehrerer Abstandselemente die Steifigkeit des Implantats angepasst werden, so dass auch durch physiologische Veränderungen im Bewegungssegment eine optimale Entlastung erreicht werden kann. Praktisch bedeutet dies, dass eine Elastizität des Abstandselements vorzugsweise so angepasst wird, dass sie mit zunehmender Druckbelastung abnimmt, was zu einer Versteifung und somit einer größeren Stabilisierung des Bewegungssegments führt. Beispielsweise kann durch Entlastung das hydrierbare Abstandselement wieder Wasser aufnehmen, wodurch es beispielsweise wieder elastischer werden kann, was eine Beweglichkeit des Bewegungssegments verbessert. Mit derartigen Implantaten ist es daher möglich, eine weitere Degenerierung einer geschädigten Bandscheibe zu stoppen oder sogar die Bandscheibe teilweise zu regenerieren. Dies kann insbesondere durch eine wieder verbesserte Hydration der Bandscheibe erreicht werden infolge einer Entlastung derselben durch das erfindungsgemäße Implantat. Das mindestens eine hydrierbare Abstandselement ist mindestens teilweise aus einem Hydrogel hergestellt. Hydrogele eignen sich hervorragend als Abstandselemente, welche infolge von physiologischen Veränderungen im Bewegungssegment sowohl ihre Form als auch ihre elastischen Eigenschaften ändern können. Dehydrierte Hydrogele sind wenig elastisch, hydrierte Hydrogele weisen dagegen eine hohe Elastizität auf. Ferner können Hydrogele je nach Aufbau irreversibel oder auch reversibel hydriert werden. Beispiele für Hydrogele sind aus der US 6,232,406 sowie der US 5,824,093 bekannt. Hydrogele werden insbesondere definiert als weiche, elastische, in Wasser quellbare, aber nicht lösliche, hydrophile polymere Materialien, deren Wassergehalt mindestens 20 Gewichtsprozent beträgt. Sie werden in zwei Unterklassen eingeteilt, nämlich degradierbare Hydrogele und nicht degradierbare Hydrogele. Degradierbare Hydrogele umfassen funktionelle Gruppen, die hydrolytisch oder enzymatisch spaltbar sind. Nicht degradierbare Hydrogele bestehen in der Regel aus Kohlenstoffketten, die im Körper nicht abbaubar sind. Vorzugsweise ist das erfindungsgemäße Implantat aus einem nicht degradierbaren Hydrogel hergestellt. Beispiele nicht degradierbarer Hydrogele sind hochmolekularer Polyvinylalkohol (PVAL), Poly-2-hydroxyethylmethacrylate (PHEMA), Acrylat Copolymere sowie hydrophiles Polyurethan. Grundsätzlich wäre es denkbar, dass das mindestens eine Abstandselement isotrop verformbar ist. Das mindestens eine Abstandselement ist jedoch anisotrop verformbar, so dass es seine Form und/oder ein umschlossenes Volumen infolge einer Änderung mindestens einer Umgebungsbedingung anisotrop ändert. Auf diese Weise lassen sich gezielt Einschränkungen einer Beweglichkeit des Bewegungssegments mit dem erfindungsgemäßen Implantat einstellen, und zwar zum Beispiel abhängig von physiologischen Veränderungen am Bewegungssegment. Bei erfindungsgemäß Verwendung eines Hydrogels ist es auf einfache Weise möglich, dass das mindestens eine anisotrop verformbare Abstandselement seine Form und/oder ein umschlossenes Volumen infolge einer Hydrierung anisotrop ändert.

Besonders einfach wird der Aufbau des Implantats, wenn das Abstandselement aus einem Formgedächtniswerkstoff hergestellt ist. Damit lassen sich gezielt Formänderungen aufgrund unterschiedlicher Umgebungsbedingungen hervorrufen, insbesondere Formänderungen infolge von Temperaturänderungen und/oder einwirkender Kräfte.

Vorzugsweise ist der Formgedächtniswerkstoff ein Metall oder ein Kunststoff. Als Metalle eignen sich insbesondere Nickel-Titan (NiTi), das auch als Nitinol bezeichnet wird, Kupfer-Zink (CuZn), Kupfer-Zink-Aluminium (CuZnAl), Kupfer-Zink-Nickel (CuZnNi) oder Eisen-Nickel-Aluminium (FeNiAl). Des Weiteren können Kunststoffe, beispielsweise Formgedächtnis-Polymere zum Einsatz kommen. Diese können derart ausgebildet sein, dass sie zum Beispiel infolge einer Erwärmung ihre ursprüngliche Form wieder einnehmen. Ein Beispiel für einen Kunststoff, der einen Einweg-(Memory)-Effekt zeigt, ist ein sogenannter "Memory-Schaum" ("memory foam"), der aus einem Polyurethan unter Zusatz weiterer Chemikalien hergestellt ist. Es wird beispielsweise zur Herstellung von Matratzen verwendet. Des Weiteren können Formgedächtnis-Polymere auch einen umkehrbaren Formgedächtniseffekt aufweisen, der nicht thermisch, sondern optisch gesteuert wird. Denkbar sind hier insbesondere Butylacrylate, die an ihren Seitenketten über Zimtsäure-Gruppen unter Bestrahlung von ultraviolettem Licht einer dafür geeigneten Wellenlänge vernetzen und die Bindung bei Bestrahlung mit Licht einer anderen Wellenlänge wieder lösen. Eine einseitige Bestrahlung eines Bauteils kann so zu einer einseitigen Vernetzung und folglich anisotropen Formänderung führen. Des Weiteren sind auch magnetisch induzierte Formgedächtnis-Polymere denkbar.

Um das Implantat möglichst einfach und vorzugsweise durch einen minimalinvasiven Zugang in einen menschlichen oder tierischen Körper einführen zu können, ist es günstig, wenn das mindestens eine Abstandselement in einer Implantationsstellung des Implantats, in welcher es in einen menschlichen oder tierischen Körper einführbar ist, maximal dehydriert ist.

Günstigerweise ist das mindestens eine Abstandselement derart ausgebildet, dass es aufgrund osmotischen Drucks im extrazellulären Raum eines menschlichen oder tierischen Körpers hydrierbar ist. Das Implantat kann sich so durch Änderungen des Abstandselements beispielsweise an physiologische Änderungen anpassen. Es kann sich insbesondere mit im extrazellulären Raum vorhandenem Wasser anreichern und so seine Form und/oder seine elastischen Eigenschaften ändern.

Gemäß einer bevorzugten Ausführungsform der Erfindung kann vorgesehen sein, dass das mindestens eine Abstandselement derart ausgebildet ist, dass es infolge einer Druckeinwirkung mindestens teilweise dehydrierbar ist. Vorzugsweise ist es vollständig dehydrierbar, wobei unter vollständiger Dehydration, beispielsweise eines Hydrogels, zu verstehen ist, dass es nur so viel Wasser verliert, dass es auch wieder Wasser aufnehmen kann. Eine mindestens teilweise Dehydrierung infolge einer Druckeinwirkung ermöglicht es, das Implantat so auszugestalten, dass das Abstandselement beispielsweise infolge einer Druckeinwirkung weniger elastisch wird und somit ein geschädigtes Bewegungssegment der Wirbelsäule stärker stabilisiert. Wird die Belastung vom Abstandselement wieder entfernt, so kann das Abstandselement auch wieder Wasser aufnehmen und wiederum seine Form und/oder seine elastischen Eigenschaften ändern.

Um das Einsetzen und Festlegen des Implantats an der Wirbelsäule zu erleichtern, ist es vorteilhaft, wenn das Implantat von einer Implantationsstellung, in welcher es an mindestens einem Dornfortsatz eines Wirbels der Wirbelsäule anlegbar und/oder von diesem lösbar ist, in eine Stabilisierungsstellung bringbar ist, in welcher es an dem mindestens einen Dornfortsatz festlegbar ist. Vorzugsweise ist das Implantat in der Implantationsstellung so geformt, dass es durch einen minimalinvasiven Zugang in einen Körper eingeführt werden kann.

Damit eine dauerhafte und stabile Festlegung des Implantats an Wirbeln einer Wirbelsäule erreicht werden kann, ist es vorteilhaft, wenn das Implantat mindestens eine Dornfortsatzaufnahme aufweist, in und/oder an welcher in der Stabilisierungsstellung mindestens einer von zwei Dornfortsätzen zweier benachbarter Wirbel gehalten ist. Es könnten auch zwei Dornfortsätze in der mindestens einen Dornfortsatzaufnahme gehalten sein, das heißt diese könnte insbesondere so groß sein, dass zwei Dornfortsätze in diese einführbar sind, beispielsweise in der Implantationsstellung.

Um auf einfache Weise eine sichere Verbindung zwischen dem Implantat und einem Dornfortsatz zu ermöglichen, ist es günstig, wenn die mindestens eine Dornfortsatzaufnahme in der Implantationsstellung geöffnet und in der Stabilisierungsstellung ringförmig geschlossen ist. Ein derart ausgebildetes Implantat kann insbesondere ohne Zuhilfenahme weiterer Befestigungsmittel, wie zum Beispiel Knochenschrauben oder Knochennägel, an Wirbeln oder an Teilen derselben festgelegt werden. Beispielsweise kann die Dornfortsatzaufnahme einen Dornfortsatz ringförmig umschließen und an diesem verspannt werden.

Vorzugsweise weist das mindestens eine Abstandselement mindestens eine Dornfortsatzanlageflächenbereich auf und ist derart ausgebildet, dass der Dornfortsatzanlageflächenbereich in der Stabilisierungsstellung an mindestens einem Dornfortsatz anliegt. So ist es möglich, dass ein Dornfortsatz direkt an dem mindestens einen Abstandselement anliegt und durch dieses in seiner Bewegung eingeschränkt wird. Das Abstandselement kann insbesondere aber auch die Befestigungsvorrichtung am Dornfortsatz verspannen, und zwar zum Beispiel infolge einer Form- und/oder Volumenänderung.

Vorteilhafterweise sind zwei Dornfortsatzaufnahmen vorgesehen. Dies ermöglicht es, jeden Dornfortsatz des Bewegungssegments separat in einer Dornfortsatzaufnahme festzulegen. Dadurch wird zum einen eine Stabilität des Implantats erhöht und zum anderen ein dauerhafter Einsatz des Implantats verbessert.

Gemäß einer bevorzugten Ausführungsform der Erfindung kann vorgesehen sein, dass eine freie Querschnittsfläche der mindestens einen Dornfortsatzaufnahme mit zunehmender Hydrierung des mindestens einen Abstandselements kleiner wird. Eine Verringerung der Querschnittsfläche führt bei einem in dem mindestens einen Dornfortsatzaufnahme eingeführten Dornfortsatz vorzugsweise dazu, dass die Befestigungsvorrichtung am Dornfortsatz verspannt wird. Günstig ist es, wenn das mindestens eine Abstandselement zwei Dornfortsatzaufnahmen voneinander trennt. Dies bedeutet insbesondere, dass das mindestens eine Abstandselement in einer Implantationsstellung zwischen zwei Dornfortsätzen angeordnet sein kann. Dadurch kann das mindestens eine Abstandselement direkt oder indirekt einen Abstand zwischen den beiden Dornfortsätzen in gewünschter Weise vorgeben.

Um sowohl in Extension als auch Flexion eines Bewegungssegments der Wirbelsäule die vorteilhaften Eigenschaften des Implantats nutzen zu können, ist es günstig, wenn mindestens zwei Abstandselemente vorgesehen sind, die an einer Dornfortsatzaufnahme einander diametral gegenüberliegend angeordnet sind. Insbesondere können die Abstandselemente so angeordnet sein, dass ein in der Dornfortsatzaufnahme gehaltener Dornfortsatz bei einer Flexion des Bewegungssegments gegen das eine Abstandselement drückt und bei einer Extension des Bewegungssegments gegen das andere Abstandselement.

Günstigerweise sind drei Abstandselemente vorgesehen. Insbesondere kann eines so angeordnet sein, dass es nach der Implantation des Implantats zwischen zwei Dornfortsätzen liegt oder gehalten ist, die beiden anderen Abstandselemente vorzugsweise dem zwischen den Dornfortsätzen angeordneten Abstandselement jeweils diametral gegenüberliegend an zwei zur Aufnahme der Dornfortsätze vorgesehenen Dornfortsatzaufnahmen. Je nach Ausführung der Befestigungsvorrichtung kann so eine gewünschte dynamische Stabilisierung des betroffenen Bewegungssegments der Wirbelsäule erreicht werden.

Um das Implantat insbesondere durch einen minimalinvasiven Zugang leicht in einen menschlichen oder tierischen Körper einführen zu können, ist es vorteilhaft, wenn das mindestens eine Abstandselement in einer Grundstellung ein Volumen aufweist, welches mindestens 3mal kleiner ist als ein Volumen des Abstandselements in einer maximalen Expansionsstellung. Ferner kann mit einem solchen Abstandselement eine Stabilisierung eines zu behandelnden Bewegungssegments der Wirbelsäule in praktisch jeder gewünschten Form erreicht werden.

Vorzugsweise ist das mindestens eine Abstandselement in der Grundstellung vollständig dehydriert und in der maximalen Expansionsstellung vollständig hydriert. Ein Übergang zwischen der Grundstellung und der maximalen Expansionsstellung kann dann durch entsprechende Hydrierung oder Dehydrierung des mindestens einen Abstandselements erreicht werden.

Der Aufbau des Implantats vereinfacht sich insbesondere dann, wenn das mindestens eine Abstandselement symmetrisch geformt ist. Es kann sowohl in der Implantationsstellung als auch in der Stabilisierungsstellung symmetrisch geformt sein. Möglich ist es jedoch auch, dass das mindestens eine Abstandselement nur in einer der beiden genannten Stellungen symmetrisch geformt ist.

Vorteilhafterweise ist das mindestens eine Abstandselement derart ausgebildet und an der Befestigungsvorrichtung angeordnet, dass es eine Bewegung miteinander verbundener Dornfortsätze aufeinander zu begrenzt. Dies kann beispielsweise dadurch erreicht werden, dass das mindestens eine Abstandselement in der Implantationsstellung zwischen zwei Dornfortsätze angeordnet wird.

Grundsätzlich wäre es denkbar, das Abstandselement einstückig auszubilden. Vorteilhafterweise umfasst das mindestens eine Abstandselement jedoch mindestens zwei Abstandselementteile. Ein solches Abstandselement ermöglicht es insbesondere auf einfache Weise, eine anisotrope Formänderung infolge geänderter Umgebungsbedingungen zu erreichen. Auch wäre es möglich, Abstandselementteile aus unterschiedlichen Materialien zu bilden, die auf geänderte Umgebungsbedingungen unterschiedlich reagieren, insbesondere ihre Form und/oder ihre elastischen Eigenschaften unterschiedlich ändern. Dadurch können noch gezielter gewünschte Stabilisierungs- und/oder Beweglichkeitsanforderungen mit dem Implantat eingestellt werden.

Vorzugsweise sind die mindestens zwei Abstandselementteile unterschiedlich anisotrop hydrierbar, so dass sie ihre Form und/oder ein umschlossenes Volumen infolge einer Hydrierung anisotrop ändern. Unterschiedlich anisotrop bedeutet insbesondere auch, dass die mindestens zwei Abstandselementteile zwar identisch ausgebildet und damit auch identisch anisotrop hydrierbar sind, jedoch aufgrund unsymmetrischer Anordnung sich insgesamt eine Anisotropie infolge einer Hydrierung oder Dehydrierung des Abstandselements ergibt.

Vorzugsweise sind die mindestens zwei Abstandselementteile derart geformt und miteinander verbunden, dass sie infolge einer Hydrierung eine Form und/oder ein umschlossenes Volumen in zwei voneinander linear unabhängigen Richtungen ändern. Beispielsweise kann so die Befestigungsvorrichtung in unterschiedlichen Richtungen relativ zu einem Dornfortsatz gezielt verspannt werden.

Besonders einfach wird der Aufbau des Abstandselements, wenn die mindestens zwei Abstandselementteile schichtförmig übereinander angeordnet sind.

Vorzugsweise sind die mindestens zwei schichtförmig übereinander angeordneten Abstandselementteile durch Schichten höherer Steifigkeit getrennt. Dadurch kann in gewünschter Weise eine Anisotropie der Form- und/oder Volumen- und/oder Elastizitätseigenschaftsänderung des Abstandselements erreicht werden.

Um die Stabilität der Abstandselemente zu erhöhen, insbesondere eine Abriebfestigkeit derselben für den Fall, dass sie direkt oder indirekt am Bewegungsapparat des Körpers angreifen, ist es vorteilhaft, wenn die Schichten höherer Steifigkeit Gewebeschichten und/oder faserverstärkte Schichten umfassen.

Auf einfache Weise lässt sich ein Abstandselement ausbilden, welches eine Anisotropie seiner Eigenschaften in zwei zueinander unabhängigen Richtungen aufweist, wenn ein erster Abstandselementteil einen Kern des Abstandselements bildet und wenn ein zweiter Abstandselementteil das erste Abstandselementteil mindestens abschnittsweise ringförmig umgibt. Beispielsweise kann das eine Abstandselementteil in Form eines zylindrischen Stabes ausgebildet sein, welches von einem zweiten hülsenartigen Abstandselementteil umgeben ist. Es wäre auch denkbar, das erste Abstandselementteil in Form einer Fadenrolle auszubilden und das zweite Abstandselementteil, anstelle eines aufgewickelten Fadens bei einer Fadenrolle, um einen zylindrischen Kern des ersten Abstandselementteils anzuordnen.

Besonders einfach wird der Aufbau des mindestens einen Abstandselements, wenn es kissenförmig ausgebildet ist.

Um eine ideale Krafteinleitung in das mindestens eine Abstandselement zu ermöglichen und einen Abrieb desselben bei Kontakt mit dem Bewegungsapparat zu reduzieren, ist es günstig, wenn das mindestens eine Abstandselement eine Abstandselementhülle und einen Abstandselementkern umfasst und wenn der Abstandselementkern infolge einer Änderung von Umgebungsbedingungen und/oder auf ihn einwirkender Kräfte seine äußere Gestalt und/oder seine elastischen Eigenschaften ändert. Die Abstandselementhülle dient somit in erster Linie dem Schutz des Abstandselementenkerns. Sie kann jedoch auch eine weitere Funktion haben, nämlich eine Form des Abstandselements vorzugeben, vorzugsweise in einem maximal expandierten Zustand. Die Abstandselementhülle kann wahlweise inelastisch oder elastisch ausgebildet sein.

Vorzugsweise ist die Abstandselementhülle aus einem Gewebe hergestellt. Ein Gewebe ermöglicht insbesondere das Durchdringen mit Flüssigkeiten, insbesondere mit Wasser. Dies ist insbesondere dann wichtig, wenn der Abstandselementkern aus einem hydrierbaren Material hergestellt ist.

Um die Stabilität der Abstandselementhülle zu erhöhen, ist diese günstigerweise faserverstärkt.

Besonders einfach wird der Aufbau des Implantats, wenn die Befestigungsvorrichtung mindestens ein Band umfasst. Beispielsweise können die Befestigungsvorrichtungen in Form eines ringförmig geschlossenen Bandes ausgebildet sein, das sich optional zum Anlegen an zwei Dornfortsätze benachbarter Wirbel öffnen lässt.

Um das Implantat dauerhaft und sicher an Dornfortsätzen benachbarter Wirbel festlegen zu können, ist das mindestens eine Band in der Stabilisierungsstellung gekreuzt und in Form einer Doppelschlaufe verlaufend ausgebildet. Das Band umschlingt somit in der Stabilisierungsstellung den einen und den anderen Dornfortsatz, wobei sich bei einem einfachen Überkreuzen des Bandes ein Umlaufsinn des Bandes um die beiden Dornfortsätze ändert, bei einem doppelt gekreuzten Band der Umlaufsinn des Bandes um die Dornfortsätze gleich bleibt. Insbesondere kann dort, wo sich das Band einfach oder mehrfach kreuzt, das mindestens eine Abstandselement angeordnet sein. Vorzugsweise ist das Band am mindestens einen Abstandselement festgelegt.

Um das Implantat einfach und sicher in einen menschlichen oder tierischen Körper einbringen zu können, ist das mindestens eine Band aus einem gewebten Material hergestellt. So ist es insbesondere ausreichend flexibel, um auch minimalinvasiv implantiert zu werden.

Gemäß einer bevorzugten Ausführungsform der Erfindung kann die Befestigungsvorrichtung in Form einer Zuggurtung ausgebildet sein, welche eine Bewegung miteinander verbundener Dornfortsätze voneinander weg begrenzt. Eine solche Zuggurtung lässt insbesondere eine Bewegung miteinander verbundener Dornfortsätze aufeinander hin zu, wobei die Bewegung jedoch wiederum durch das mindestens eine Abstandselement begrenzt werden kann.

Vorteilhaft ist es, wenn die Befestigungsvorrichtung mindestens eine Verschlussvorrichtung aufweist und wenn mit der mindestens einen Verschlussvorrichtung die mindestens eine Dornfortsatzaufnahme in der Implantationsstellung öffenbar und in der Stabilisierungsstellung ringförmig verschließbar ist. Die Verschlussvorrichtung ermöglicht es auf einfache Weise, beispielsweise ein von der Befestigungsvorrichtung umfasstes Band ringförmig zu schließen oder zu Implantationszwecken zu öffnen.

Günstigerweise ist die Verschlussvorrichtung in Form einer unidirektionalen Verschlussvorrichtung ausgebildet, insbesondere in Form eines Kabelbinderverschlusses. Dies gestattet es, beispielsweise eine ringförmig geöffnete Befestigungsvorrichtung, zum Beispiel in Form eines Bandes, zu implantieren und um einen Dornfortsatz zu schlingen. Ein Schließen und Verspannen kann mit einer unidirektionalen Verschlussvorrichtung in einem Schritt erfolgen, und zwar durch einfaches Ziehen.

Alternativ oder zusätzlich kann es vorteilhaft sein, wenn die Verschlussvorrichtung eine Pressklemme umfasst. Dadurch kann eine besonders sichere Verbindung der beiden miteinander zu verbindenden Teile zum Schließen der Befestigungsvorrichtung erreicht werden.

Zur Erhöhung der Stabilität des Implantats ist es günstig, wenn das mindestens eine Abstandselement mit der Befestigungsvorrichtung unlösbar verbunden ist.

Die eingangs gestellte Aufgabe wird bei einem Implantatsystem der eingangs beschriebenen Art erfindungsgemäß dadurch gelöst, dass das Implantat ein Implantat nach einem der Ansprüche 1 bis 14 ist.

Ein derartiges Implantatsystem ermöglicht es insbesondere, ein geschädigtes Bewegungssegment einer menschlichen oder tierischen Wirbelsäule dynamisch und dorsal zu stabilisieren, wie das insbesondere oben bereits ausführlich beschrieben wurde. Das Implantatsystem umfasst vorzugsweise ein oder mehrere Instrumente, mit deren Hilfe das Implantat eingesetzt und auf einfache Weise an Dornfortsätzen benachbarter Wirbel festgelegt werden kann. Als Instrumente sind insbesondere Skalpelle, Klemmen, Scheren, Nadelhalter, Nadeln mit Fäden und dergleichen denkbar. Alle Instrumente können insbesondere auch als Rohrschaftinstrumente, also für einen minimalinvasiven oder perkutanen Zugang.

Um das Implantatsystem in gewünschter Weise an jeweils vorliegende Erfordernisse im Zusammenhang mit einer dynamischen dorsalen Stabilisierung eines Bewegungssegments einer menschlichen oder tierischen Wirbelsäule anpassen zu können, ist es vorteilhaft, wenn das Implantat eines der oben beschriebenen Implantate ist.

Des Weiteren wird ein Verfahren zur dorsalen Stabilisierung einer menschlichen oder tierischen Wirbelsäule mit einem der oben beschriebenen Implantate oder einem der oben beschriebenen Implantatsysteme offenbart, bei welchem
- ein Zugang zum menschlichen oder tierischen Körper eröffnet wird und
- das Implantat mit mindestens teilweise dehydriertem Abstandselement in den Körper eingebracht und die Befestigungsvorrichtung an Dornfortsätze zweier benachbarter Wirbel angelegt und mindestens lose mit diesem verbunden wird.

Dieses einfache Verfahren ermöglicht unter Verwendung der beschriebenen Implantate beziehungsweise Implantatsysteme eine dynamische Stabilisierung eines Bewegungssegments einer Wirbelsäule.

Gemäß einer bevorzugten Variante des Verfahrens wird ein minimalinvasiver Zugang eröffnet. Auf diese Weise kann ein Operationstrauma minimiert und ein Heilungsprozess nach der Implantation deutlich verbessert werden.

Die nachfolgende Beschreibung bevorzugter Ausführungsformen der Erfindung dient im Zusammenhang mit der Zeichnung der näheren Erläuterung. Es zeigen:
- Figur 1:: ein erstes Ausführungsbeispiel an zwei Dornfortsätze benachbarter Wirbel angelegtes Implantat mit Abstandselementen in der Implantationsstellung;
- Figur 2:: eine Ansicht ähnlich Figur 1, jedoch mit Abstandselementen in der Stabilisierungsstellung;
- Figur 3:: eine perspektivische Ansicht des Implantats von dorsal aus Figur 1 in der Implantationsstellung;
- Figur 4:: eine perspektivische Rückansicht des Implantats aus Figur 3;
- Figur 5:: eine perspektivische Ansicht eines zweiten Ausführungsbeispiels eines Implantats;
- Figur 6:: eine perspektivische Ansicht eines dritten an zwei Dornfortsätze benachbarter Wirbel angelegten Implantats mit Abstandselementen in der Stabilisierungsstellung;
- Figur 7:: eine Schnittansicht durch ein in Figur 6 dargestelltes anisotropes Abstandselement;
- Figur 8:: eine Seitenansicht des in den Figuren 6 und 7 dargestellten Abstandselements;
- Figur 9:: eine Querschnittsansicht des Abstandselements aus Figur 8 längs Linie 9-9;
- Figur 10:: eine schematische Darstellung des in Figur 6 dargestellten Implantats mit Abstandselementen in der Stabilisierungsstellung; und
- Figur 11:: eine Seitenansicht des Implantats aus Figur 6.

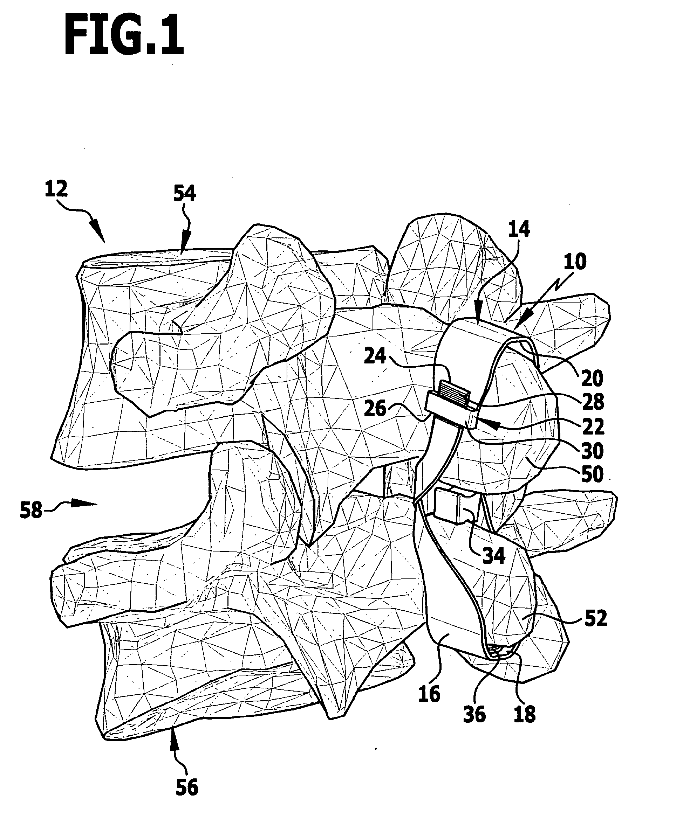

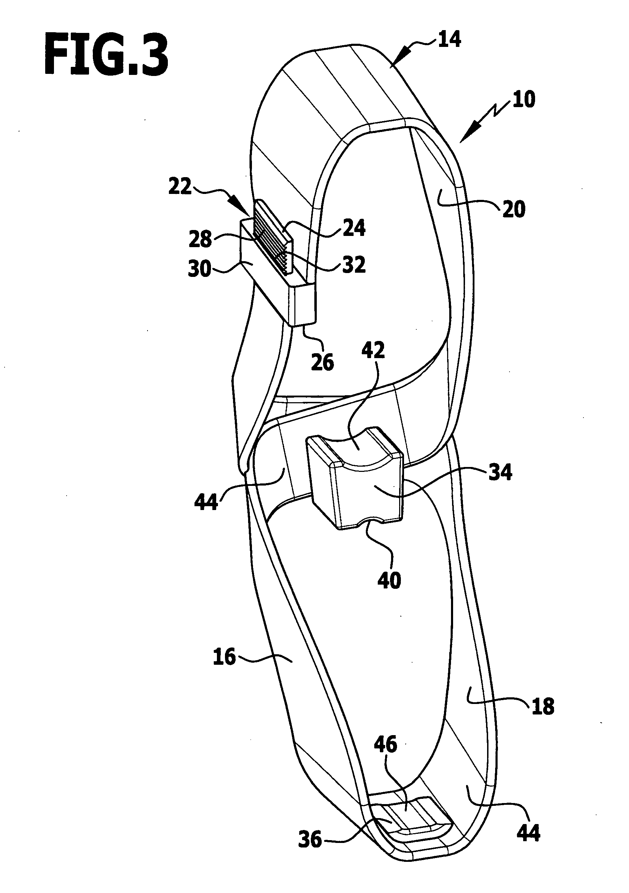

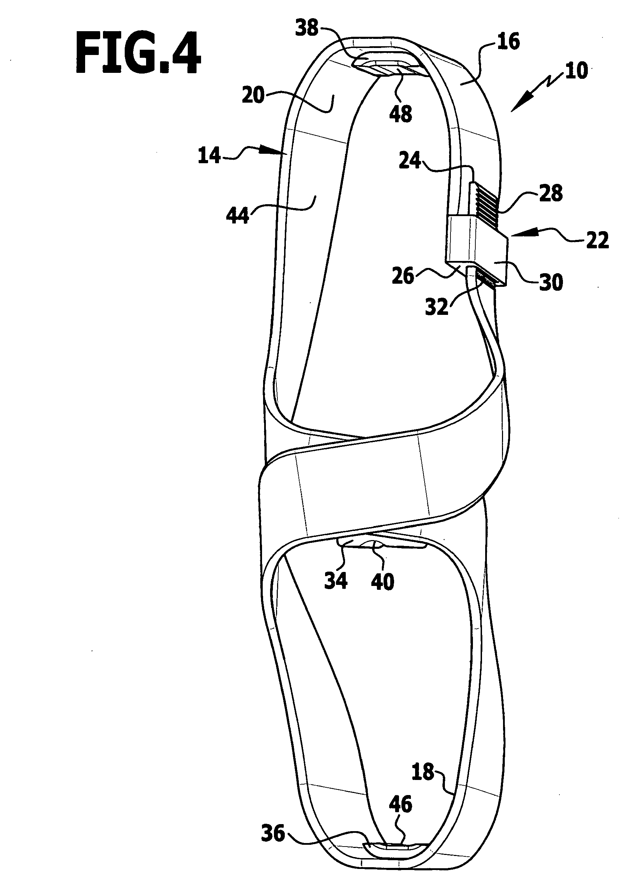

In den Figuren 1 bis 4 ist ein erstes Ausführungsbeispiel eines insgesamt mit dem Bezugszeichen 10 versehenen Implantats zur dynamischen dorsalen Stabilisierung einer menschlichen Wirbelsäule 12 dargestellt. Es umfasst eine Befestigungsvorrichtung 14 in Form eines sich kreuzenden, im Wesentlichen in Form einer "8" gelegten Bandes 16.

Das in Form einer "8" gelegte oder geschlungene Band 16 definiert zwei im Wesentlichen ringförmig geschlossene Dornfortsatzaufnahmen 18 und 20. Um das Band in sich ringförmig zu schließen ist eine Verschlussvorrichtung 22 vorgesehen, die freie Enden 24 und 26 des Bandes 16 miteinander verbindet. Bei dem in den Figuren 1 bis 4 dargestellten Ausführungsbeispiel ist die Verschlussvorrichtung 22 in Form einer unidirektionalen Verschlussvorrichtung ausgebildet, nämlich in Form eines Kabelbinderverschlusses. Zu diesem Zweck ist das freie Ende 24 einseitig mit einer quer zu einer Längsrichtung des Bandes 16 ausgebildeten Verzahnung 28 versehen. Am anderen freien Ende 26 ist ein quaderförmiger Verriegelungskörper 30 aufgesetzt, welcher eine sich in Längsrichtung erstreckende Durchbrechung 32 aufweist, in welcher ein nicht dargestelltes, in die Verzahnung 28 eingreifendes, federnd gelagertes Rastglied angeordnet ist. Die Durchbrechung 32 ist so dimensioniert, dass das freie, mit der Verzahnung 28 versehene Ende 24 durch die Durchbrechung 32 hindurchgeschoben werden kann. Durch das nicht dargestellte, in die Verzahnung 28 eingreifende Rastglied werden die freien Enden 24 und 26 relativ zueinander gesichert. Ferner kann durch ein Nachziehen des freien Endes 24 aus dem Verriegelungskörper 30 heraus eine Spannung des Bandes 16 erhöht werden, wodurch die Dornfortsatzaufnahmen 18 und 20 im Durchmesser beziehungsweise ihre freien Querschnittsflächen etwas kleiner werden.

Der Befestigungsvorrichtung 14 sind insgesamt drei Abstandselemente 34, 36 und 38 zugeordnet. Das Abstandselement 34 ist in etwa würfelförmig ausgebildet und aus einem Hydrogel hergestellt, beispielsweise einem hochmolekularen Polyvinylalkohol (PVAL). Es handelt sich dabei um ein nicht degradierbares Hydrogel. Auf einer Ober- und einer Unterseite des Abstandselements 34 sind parallel zueinander ausgerichtete rinnenförmige Vertiefungen 40 und 42 vorgesehen. Das Abstandselement 34 ist ferner auf einer Innenseite 44 des Bandes 16 befestigt. Die Vertiefungen 40 und 42 weisen in Richtung auf die Dornfortsatzaufnahmen 18 und 20 hin. Praktisch begrenzt das Abstandselement 34 die beiden Dornfortsatzaufnahmen 18 und 20, denn es ist direkt zwischen ihnen angeordnet. An gegenüberliegenden Enden der Dornfortsatzaufnahmen 18 und 20 sind die im Wesentlichen flachen quaderförmigen Abstandselemente 36 und 38 angeordnet, die ebenfalls mit flachen rinnenförmigen Vertiefungen 46 beziehungsweise 48 versehen sind, die jeweils pa rallel zu den Vertiefungen 40 und 42 des Abstandselemente 34 ausgerichtet sind. Die Vertiefungen 40, 42, 46 und 48 definieren Dornfortsatzanlageflächenbereiche, die nach Implantation des Implantats 10 mit Dornfortsätzen 50 und 52 in Kontakt stehen. Die Abstandselemente 36 und 38 sind jeweils auch aus einem Hydrogel gebildet.

Eine wesentliche Eigenschaft des Hydrogels ist es, dass es Wasser aufnehmen und dadurch sein Volumen drastisch vergrößern kann; insbesondere ist es möglich, dass ein maximal dehydriertes Hydrogel, welches noch einen Wasseranteil von etwa 20 Gewichtsprozent enthält, im dehydrierten Zustand ein Volumen aufweist, das etwa 1/10 bis 1/20 des Volumens in einem maximal hydrierten Zustand entspricht. In jedem Fall sind die Abstandselemente 34, 36 und 38 derart ausgebildet, dass sie infolge einer Änderung von Umgebungsbedingungen ihre äußere Gestalt und/oder ihre elastischen Eigenschaften ändern. Die elastischen Eigenschaften eines Hydrogels ändern sich entsprechend seiner Hydrierung. Je mehr Wasser ein Hydrogel aufgenommen hat, umso elastischer ist es. Somit ist es in seinem maximal dehydrierten Zustand am wenigsten elastisch.

Zur dynamisch dorsalen Stabilisierung eines Bewegungssegments der Wirbelsäule 12 wird das Implantat 10 an den Dornfortsätzen 50 und 52 benachbarter Wirbel 54 und 56 festgelegt. Zu diesem Zweck wird vorzugsweise ein minimalinvasiver Zugang zum Körper des Patienten eröffnet. Das noch nicht in sich geschlossene Band 16 wird dann eingeführt und von dorsal aus gesehen in Form einer "8" um die Dornfortsätze 50 und 52 gelegt. Ausgehend vom freien Ende 26 wird dieses im Uhrzeigersinn um den oberen Dornfortsatz 50 geschlungen und zwischen dem Dornfortsatz 50 und dem Dornfortsatz 52 durchgeführt. Das an der Innenseite 44 befestigte Abstandselement 34 sitzt dann genau zwischen den beiden Dornfortsätzen 50 und 52. Das Band 16 wiederum wird im Gegenuhrzeigersinn um den unteren Dornfortsatz 52 des Wirbels 56 geschlungen und kreuzt sich selbst hinter dem Abstandselement 34. Es wird dann weiter im Uhrzeigersinn mit seinem freien Ende 24 bis an das freie Ende 26 herangeführt und mittels der Verschlussvorrichtung 22 mit diesem verbunden. Die Dornfortsätze 50 und 52 durchsetzen somit die Dornfortsatzaufnahmen 20 beziehungsweise 18.

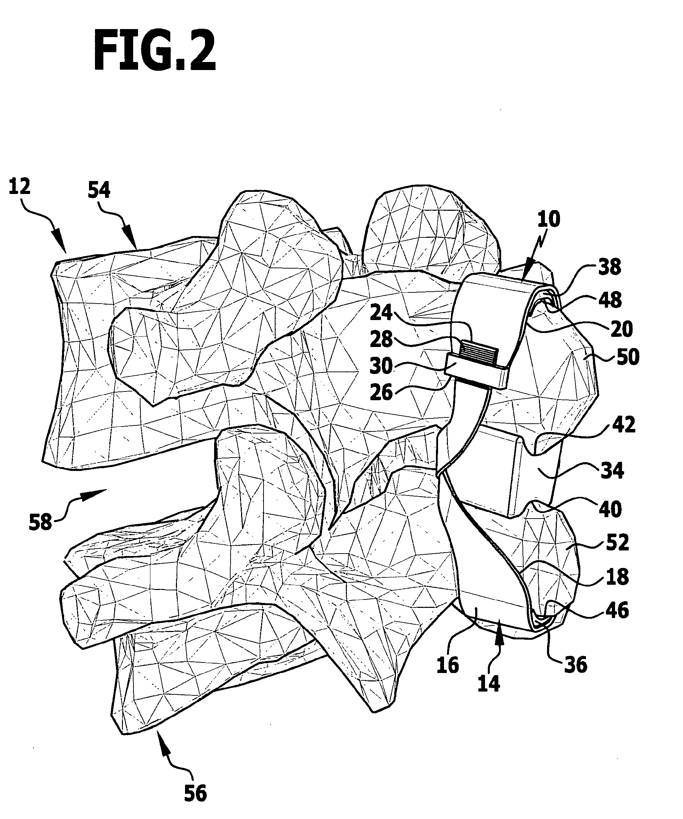

Das Band 16, das aus einem gewebten, biodegradierbaren oder auch nicht abbaubaren Material hergestellt ist, wird mittels der Verschlussvorrichtung 22 nicht maximal eng beziehungsweise fest um die Dornfortsätze 50 und 52 verspannt. Vielmehr liegt es im Wesentlichen lose um diese herum. Aufgrund eines im extrazellulären Raum des Patientenkörpers herrschenden osmotischen Drucks saugen sich die Abstandselemente nach dem Einsetzen des Implantats 10 mit Wasser voll, das heißt sie hydrieren. Ausgehend von einer Implantationsstellung, in der die Abstandselemente 34, 36 und 38 dehydriert sind, vorzugsweise maximal dehydriert, nehmen sie nach dem Hydrieren ein Volumen ein, das etwa 3mal bis 20mal größer ist als in der in den Figuren 1, 3 und 4 dargestellten Implantationsstellung des Implantats. Durch die Volumenzunahme der Abstandselemente 34, 36 und 38 wird die Befestigungsvorrichtung 14 an den Dornfortsätzen 50 und 52 verspannt. Das Implantat 10 nimmt jetzt seine in Figur 2 dargestellte Stabilisierungsstellung ein.

Aufgrund der Verwendung von Abstandselementen 34, 36 und 38, die derart ausgebildet sind, dass sie infolge einer Änderung von Umgebungsbedingungen und/oder auf sie einwirkender Kräfte ihre äußere Gestalt und/oder ihre elastischen Eigenschaften ändern, ist es möglich, mit dem Implantat 10, das durch die beiden Wirbel 54 und 56 sowie die in einem Zwischenwirbelraum 58 angeordnete, in den Figuren 1 und 2 jedoch nicht dargestellte, Bandscheibe definierte Bewegungssegment der Wirbelsäule 12 dynamisch dorsal zu stabilisieren. Ein solcher Eingriff ist beispielsweise zur Entlastung einer teilweise degenerierten Bandscheibe dienlich oder zur temporären Stabilisierung nach einer Bandscheibenoperation, beispielsweise nach einer Teilresektion derselben infolge eines Bandscheibenvorfalls.

Durch die eine Zuggurtung bildende Befestigungsvorrichtung 14 verhindert das Band 16 mit den Abstandselementen 36 und 38 eine allzu große Flexion des Bewegungssegments, das heißt eine Bewegung der Wirbel 54 und 56 aufeinander zu, bei der die Bandscheibe zusammengedrückt wird. Die hydrierten Abstandselemente 36 und 38 wirken bei einer Extensionsbewegung des Bewegungssegments als Dämpfungsglieder.

Im Gegensatz hierzu wird bei einer Extension, also einer Streckbewegung der Wirbelsäule 12, die Bandscheibe zwischen den Wirbeln 54 und 56 entlastet. Folglich bewegen sich die beiden Dornfortsätze 50 und 52 aufeinander zu und versuchen, das zwischen ihnen gehaltene Abstandselement 34 zusammenzudrücken. Das Abstandselement 34 dient einerseits als Anschlag für die Dornfortsätze 50 und 52, andererseits jedoch auch als Dämpfungselement. Aufgrund des auf es ausgeübten Drucks gibt es zumindest teilweise wieder Wasser ab und dämpft aufgrund seiner elastischen Eigenschaften im hydrierten Zustand die Bewegung der Dornfortsätze 50 und 52 aufeinander zu. Durch die teilweise Dehydrierung aufgrund des von den Dornfortsätzen 50 und 52 ausgeübten Drucks auf das Abstandselement 34 wird dieses jedoch zunehmend inelastischer, wodurch ein minimaler Abstand zwischen den beiden Dornfortsätzen 50 und 52 definiert wird. Gleichzeitig wird es aber den Abstandselementen 36 und 38 ermöglicht, Wasser aufzunehmen, so dass sie bei einer Extensionsbewegung der Wirbelsäule 12 entlastet werden, da bei einer Extensionsbewegung eine auf die Wirbelsäule 12 wirkende Druckbelastung deutlich niedriger ist als bei einer Flexionsbewegung, die ebenfalls zu einer Dehydrierung der Abstandselemente 36 und 38 führt, wodurch deren Volumen und ihre Elastizität verringert wird. Insgesamt eignet sich das Implantat 10 somit zu einer gezielten Bewegungseinschränkung des betroffenen Bewegungssegments.

Ferner ist das Implantat 10 geeignet, sich an physiologische Veränderungen im Bewegungssegment zu adaptieren. Im Verlauf eines Tages nimmt die Bandscheibenhöhe unter Belastung durch Dehydrierung ab. Folglich drücken auch die Dornfortsätze 50 und 52 verstärkt auf das Abstandselement 34, so dass dieses unter Belastung dehydriert und zunehmend steifer wird. Dadurch entlastet das Implantat 10 die Bandscheibe. Nachts kann infolge einer Hydrierung der Bandscheibe im entlasteten Zustand auch wieder eine Entlastung des Abstandselements 34 erfolgen durch eine Bewegung der Dornfortsätze 50 und 52 voneinander weg.

Vorzugsweise wird das Implantat 10, wie in den Figuren 1 und 2 dargestellt, von dorsal eingebracht, und zwar derart, dass das Abstandselement 34 ventralseitig vom Band 16 zurückgehalten wird. Dies verhindert, dass im ungünstigsten Fall das Abstandselement 34 Druck auf den Spinalkanal ausüben kann. Das Band 16 dient somit gleichzeitig als Ausdehnungsbegrenzung für das Abstandselement 34, das sich jedoch in dorsaler Richtung infolge einer Hydrierung ausdehnen kann, wie beispielsweise in Figur 2 dargestellt.

Die Abstandselemente 34, 36 und 38 können mit dem Band 16 verklebt sein oder umfassen einen Abstandselementkern aus einem Hydrogel, der in eine Tasche eingesetzt ist, die insbesondere mit dem Band 16 verklebt, vernäht oder auf beliebige andere Weise verbunden sein kann. Die Tasche, die eine Abstandselementhülle bildet, dient insbesondere dem Schutz des eigentlichen Hydrogels, welches durch direkte Anlage an den Dornfortsätzen 50 und 52 abgenutzt werden kann. Die Abstandselementhülle kann insbesondere aus einem gestrickten oder gewirkten Gewebe hergestellt sein, das vorzugsweise faserverstärkt ist. Denkbar sind hier alle möglichen Faserarten, die körperverträglich und nicht degradierbar sind. Selbstverständlich wäre es auch denkbar, degradierbare Hydrogele oder ein insgesamt aus biodegradierbaren Materialien hergestelltes Implantat zu verwenden. Dies hätte insbesondere den Vorteil, dass bei einer nur temporär gewünschten Stabilisierung des Bewegungssegments kein weiterer chirurgischer Eingriff erforderlich wäre, um das Implantat wieder zu entfernen. Als biodegradierbare Hydrogele kommen insbesondere die infrage, die in "Degradable hydrogels. Chen, Jun; Jo, Seongbong; Park, Kinam: Drug Targeting Delivery (1997), 7 (Handbook of Biodegradable Polymers), Seiten 203 bis 230" beschrieben sind. Diese Veröffentlichung wird hiermit mit ihrem gesamten Offenbarungsgehalt in die vorliegende Anmeldung mit einbezogen.

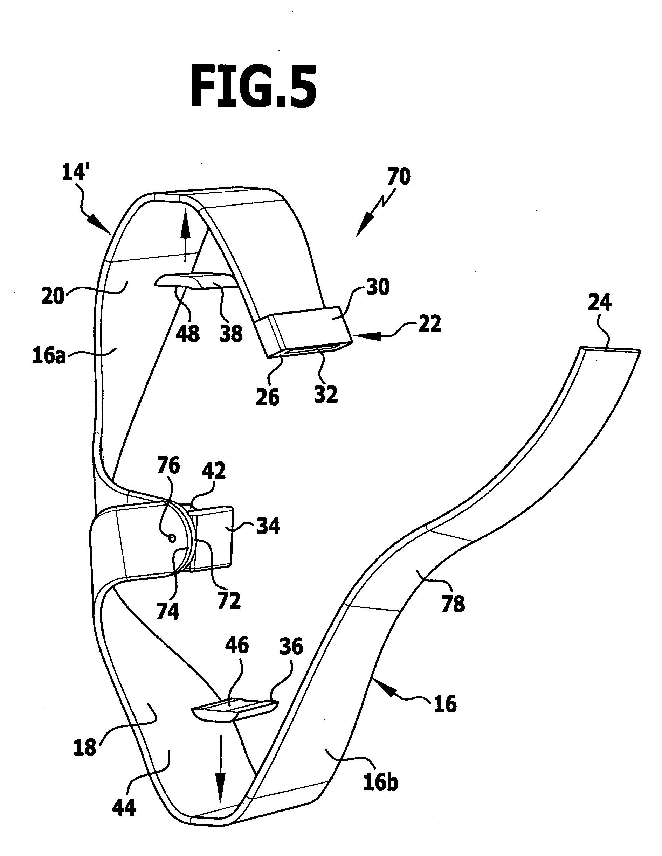

Eine Variante des Implantats 10 ist in Figur 5 dargestellt. Da es im Wesentlichen mit dem Implantat 10 übereinstimmt, werden gleiche Elemente mit denselben Bezugszeichen versehen.

Das in Figur 5 dargestellte Implantat 70 unterscheidet sich im Aufbau der Befestigungsvorrichtung 14 vom Implantat 10. Die Befestigungsvorrichtung 14' des Implantats 70 wird ebenfalls durch ein Band 16 gebildet, welches allerdings zweiteilig ausgebildet ist. Ein oberer Bandteil 16a dient zur Ausbildung einer Dornfortsatzaufnahme 20. Er weist ein erstes freies Ende 26 auf, welches mit einem Verriegelungskörper 30 versehen ist, welcher eine Durchbrechung 32 aufweist, an der ein nicht dargestellter Klemmmechanismus im Innern vorgesehen ist. Der obere Bandteil 16a weist eine Länge auf, um im Wesentlichen 270° eines Dornfortsatzes 50 zu umgeben. Auf einer Innenseite am anderen freien Ende 72 des oberen Bandteils 16a ist ein Abstandselement 34 angeordnet, welches dem Abstandselement 34 des Implantats 10 entspricht.

Das Ende 72 ist auf seiner Außenseite mit einer Innenseite eines freien Endes 74 eines unteren Bandteils 16b verbunden. Hierzu dient insbesondere ein Verbindungsstift 76 oder ein Niet. Es wäre auch denkbar, die Enden 72 und 74 miteinander zu verkleben oder zu vernähen. Der untere Bandteil 16b ist länger als der obere Bandteil 16a, so dass er den unteren Dornfortsatz 52 des Wirbels 56 vollständig umschließen kann. Er weist ferner ein zweites freies Ende 24 auf, das derart ausgebildet ist, dass es durch die Durchbrechung 32 des Verriegelungskörpers 30 eingeführt und mit diesem, zum Beispiel durch Klemmung, verbunden werden kann. Um eine ähnliche Zuggurtung zu erreichen, wie beim Implantat 10, wird vorzugsweise ein Bandabschnitt 78 des unteren Bandteils 16b, welcher nach Verbinden des freien Endes 24 mit dem freien Ende 26 in etwa auf Höhe des Abstandselements 34 angeordnet ist, entweder mit diesem verbunden oder mit den freien Enden 72 und/oder 74 des oberen Bandteils 16a beziehungsweise des unteren Bandteils 16b. Nach Verbinden des Bandabschnitts 78 beispielsweise mit den freien Enden 72 und/oder 74 weist das Implantat 70 eine Form auf, die im Wesentlichen der des Implantats 10 entspricht. Durch das Abstandselement 34 getrennt sind obere und untere Dornfortsatzaufnahmen 20 und 18, die zudem, dem Abstandselement 34 gegenüberliegend, durch auf einer Innenseite 44 des Bands 16 angeordnete Abstandselemente 36 und 38 begrenzt werden, die entsprechend den Abstandselementen 36 und 38 des Implantats 10 ausgebildet sind. Dadurch ergibt sich insgesamt eine Funktionalität des Implantats 70, die der des Implantats 10 entspricht, so dass auf die obige Beschreibung verwiesen werden kann.

Durch die zweiteilige Ausbildung des Bands 16 wird einerseits das Anlegen der Befestigungsvorrichtung 14 an die beiden Dornfortsätze 50 und 52 erleichtert, andererseits ist jedoch zu berücksichtigen, dass zur Ausbildung einer gewünschten Zuggurtung der Bandabschnitt 78 noch mit den freien Enden 72 und/oder 74 verbunden werden muss. Je nach gewünschter Behandlungssituation kann wahlweise das Implantat 10 oder das Implantat 70 zur dynamischen dorsalen Stabilisierung eines Bewegungssegments der Wirbelsäule 12 eingesetzt werden.

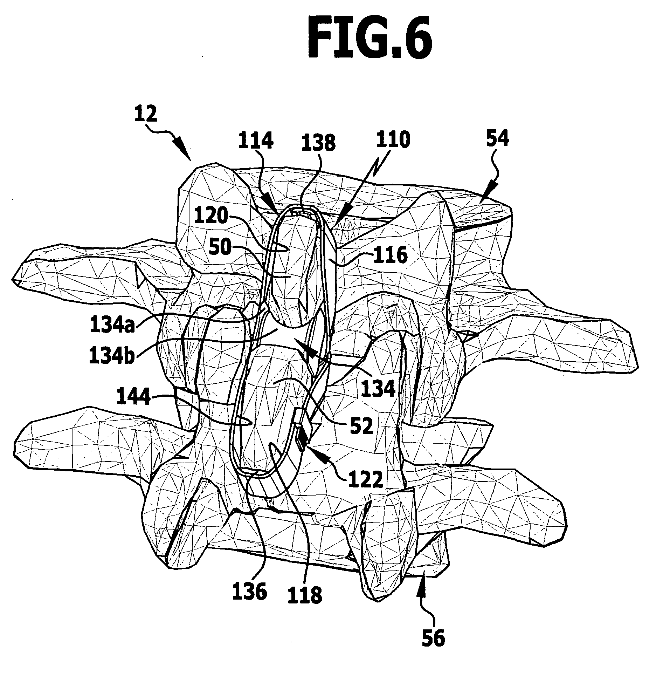

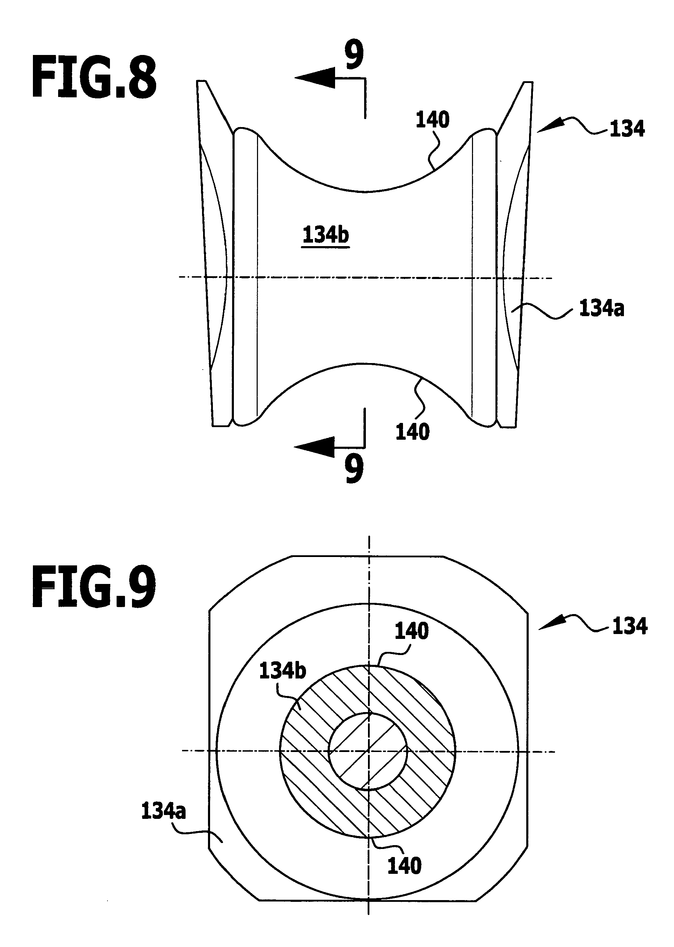

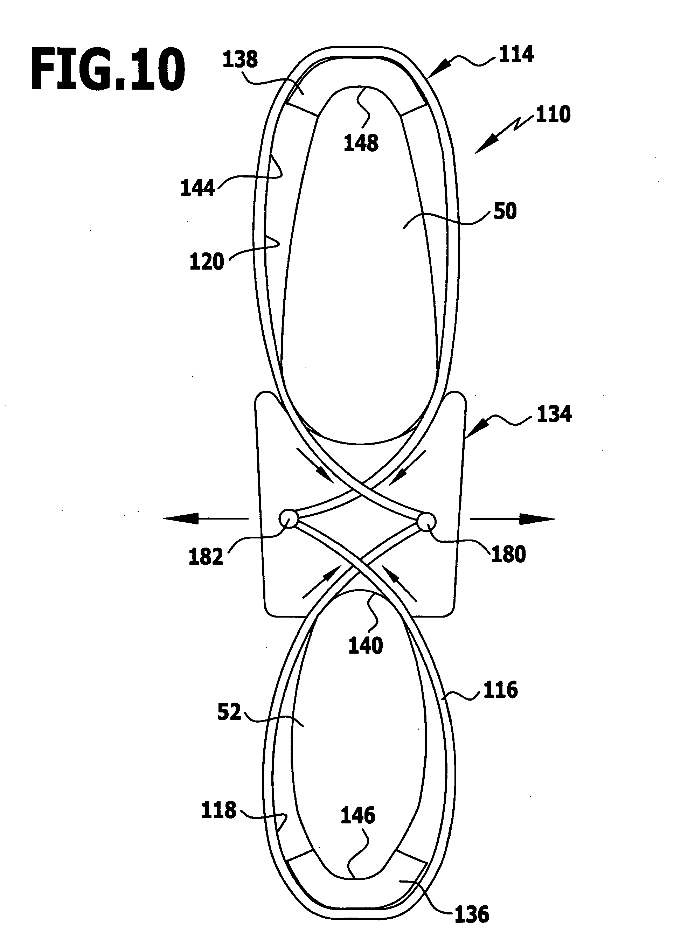

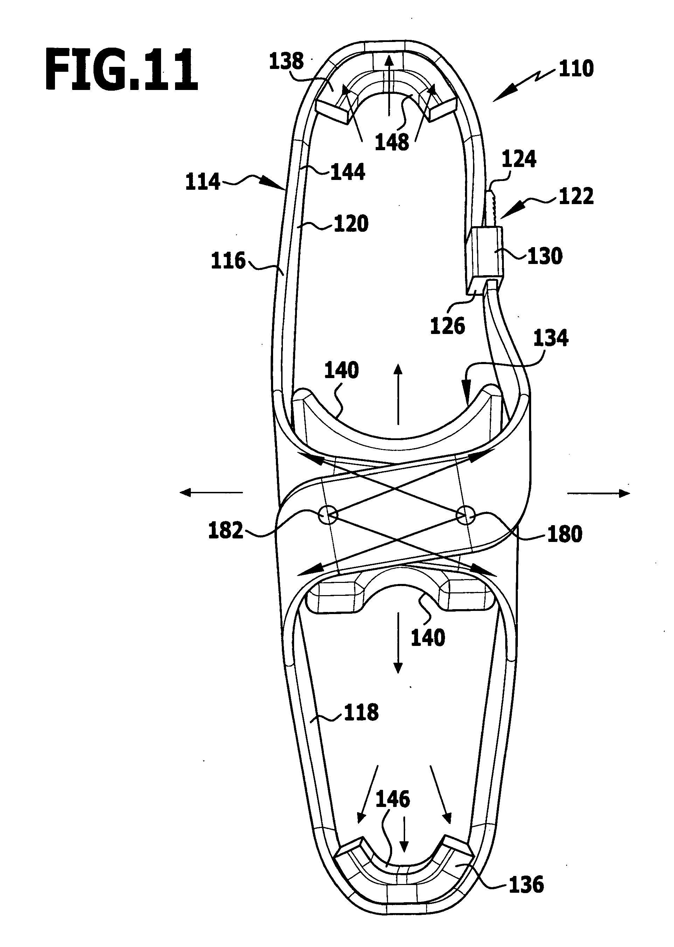

Ein drittes Ausführungsbeispiel eines Implantats zur dynamischen dorsalen Stabilisierung eines Bewegungssegments der Wirbelsäule 12 ist in den Figuren 6 bis 11 dargestellt und insgesamt mit dem Bezugszeichen 110 versehen. Teile des Implantats 110, die gleich oder im Wesentlichen gleich Teilen der Implantate 10 und 70 entsprechend ausgebildet sind, sind mit Bezugszeichen versehen, die dieselben beiden Endziffern aufweisen.

Das Implantat 110 umfasst eine Befestigungsvorrichtung 114, die im Wesentlichen der Befestigungsvorrichtung 14 entspricht. Diese umfasst ein Band 116, welches mit seiner Innenseite 144 die Dornfortsätze 50 und 52 umschlingend an diesen festgelegt werden kann. Allerdings ist das Band 116, anders als das Band 16, an zwei Befestigungspunkten 180 und 182 an einem zentralen Abstandselemente 134 festgelegt. Ein oberer Teil des Bandes 116 umläuft ausgehend vom Befestigungspunkt 180 von dorsal aus gesehen den Dornfortsatz 50 im Gegenuhrzeigersinn bis zum Befestigungspunkt 182, der am Abstandselement 134 auf gleicher Höhe wie der Befestigungspunkt 180 vorgesehen, jedoch von diesem beabstandet ist. Daher kreuzt sich das Band 116 im Bereich des Abstandselements 134 etwas oberhalb zwischen den beiden Befestigungspunkten 180 und 182. Der weitere Bandverlauf geht aus vom Befestigungspunkt 182 und umläuft den Dornfortsatz 52 ebenfalls im Gegenuhrzeigersinn bis zum Befestigungspunkt 180. Folglich kreuzt sich das Band 116 nochmals, und zwar zwischen den Befestigungspunkten 180 und 182 etwas unterhalb derselben.

Analog den Abstandselementen 36 und 38 sind auch am Implantat 110 Abstandselemente 136 und 138 an den Dornfortsatzaufnahmen 118 und 120 dem Abstandselement 134 diametral gegenüberliegend angeordnet. Das Abstandselement 134 unterscheidet sich vom Abstandselement 34 dadurch, dass es zweiteilig ausgebildet ist. Ein erster Abstandselementteil 134 weist im Wesentlichen die Form einer Fadenrolle auf, das heißt einen zylindrischen Kern mit sich daran beidseitig anschließenden konischen Erweiterungen. Anstatt von einem Faden ist das Abstandselementteil 134a von einem zweiten Abstandselementteil 134b umgeben, welches im Wesentlichen hülsenförmig ausgebildet ist. Durch die Form des Abstandselementteils 134a ist das Abstandselementteil 134b mit einer umlaufenden Vertiefung 140 versehen, an der sich die Dornfortsätze 50 und 52 anschmiegen können. Durch die zweiteilige Ausgestaltung und aufgrund deren Form ergibt sich insgesamt ein anisotropes Abstandselement 134. Da die Abstandselementteile 134a und 134b vorzugsweise aus einem Hydrogel hergestellt sind, kann die anisotrope Struktur des Abstandselements 134 insbesondere bei einer Expansion und Kompression des Abstandselements 134 zur dynamischen Anpassung des durch das Implantat 110 gekoppelten Bewegungssegments an die gesamte Wirbelsäule 12 genutzt werden.

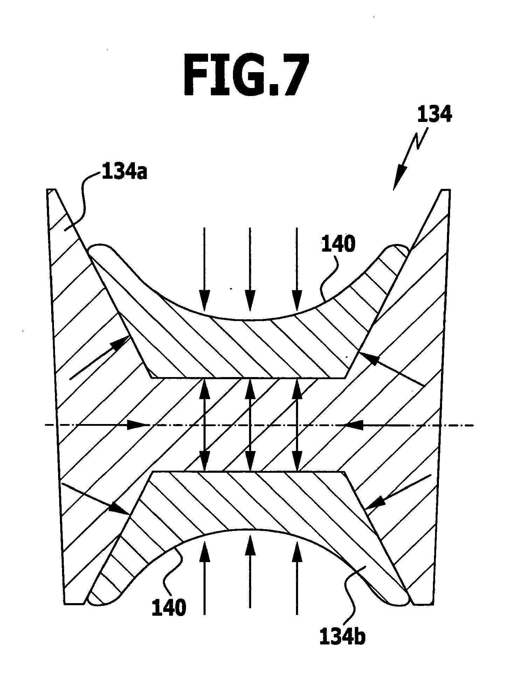

Folge der fadenrollenartigen Ausgestaltung des Abstandselementteils 134a ist, dass eine radiale Kompression desselben in einer axialen Längenänderung resultiert, wie es durch die Pfeile in Figur 7 angedeutet ist. Eine solche Längenänderung, zum Beispiel eine Verkürzung, die als "Muskelfunktion" bezeichnet werden kann, wird zum Erhalt eines dynamischen Gleichgewichts der auf das Bewegungssegment wirkenden Kräfte verwendet. Praktisch führt dies dazu, dass durch eine zunehmende Extension das Abstandselementteil 134b und das Abstandselementteil 134a komprimiert werden. Falls sie beide aus einem Hydrogel hergestellt sind, dehydrieren sie teilweise unter den wirkenden Kompressionskräften. Ist beispielsweise eine radiale Kompression des Abstandselementteils 134a durch Ausbildung einer anisotropen Struktur desselben behindert, so resultiert diese in einer axialen Verkürzung des Abstandselementteils 134a. Diese Verkürzung wiederum wirkt als seitliche Kompression auf das Abstandselementteil 134b und somit einer Extension des Bewegungssegments entgegen.

Anstelle eines zweiteiligen Abstandselements 134 kann auch ein einteiliges Abstandselement verwendet werden, welches durch entsprechenden anisotropen Aufbau eine axiale Verkürzung durch eine radiale Kompression ermöglicht.

Der doppelt gekreuzte Verlauf des Bands 116 hat bei einer Flexion des Bewegungssegments folgende Wirkung: Durch eine Zunahme der Flexion wird eine Spannung des Bands 116 erhöht, die auf das gesamte Implantat 110 wirkt. Da die Dornfortsätze 50 und 52 bei einer Flexion gegen die kleineren Abstandselemente 136 und 138 drücken, dehydrieren diese zunehmend, was zu einer Abnahme einer Spannung des Bands 116 führen kann. Gleichzeitig wird jedoch das zwischen den Dornfortsätzen 50 und 52 angeordnete Abstandselement 134 entlastet, so dass es sich lateral ausdehnen kann. Aufgrund der gekreuzten Befestigung des Bands 116 am Abstandselement 134 an den Befestigungspunkten 180 und 182 bewirkt aber eine laterale Ausdehnung des Abstandselements 134 eine Zunahme einer Spannung des Bands 116. So kann sich das Implantat 110 ideal an Veränderungen des Bewegungssegments anpassen.

Die oben beschriebenen Implantate können anstatt mit drei Abstandselementen auch jeweils nur mit einem einzelnen Abstandselement ausgestattet sein, welches in der Stabilisierungsstellung zwischen den Dornfortsätzen 50 und 52 angeordnet ist. Die beschriebenen Implantate stellen zum einen eine Balance zwischen Bewegung und Stabilität des stabilisierten Bewegungssegments her und schränken eine Bewegung, insbesondere in Extension ein. Belastungen, die auf das Bewegungssegment wirken, werden teilweise durch die Abstandselemente aufgenommen. Durch eine durch die jeweilige Befestigungsvorrichtung gebildete Zuggurtung wird eine Entlastung der Bandscheibe erreicht, was insbesondere eine Regenerierung einer teilweise degenerierten Bandscheibe fördert. Des Weiteren wird durch die sogenannte kyphotische Stellung die Fläche der Foramen und des Spinalkanals der Wirbelsäule 12 vergrößert. Dies ist zum Beispiel bei Stenosen ein Mechanismus, der zur Entlastung des Rückenmarks beziehungsweise der Cauda Equina oder der Nervenwurzeln führt. Durch die Entlastung des Bewegungssegments durch die beschriebenen Implantate ist es möglich, mit diesen eine weitere Degenerierung der Bandscheibe zu stoppen oder diese sogar teilweise zu regenerieren. Insbesondere ist eine verbesserte Hydration der Bandscheibe möglich.

Die Bänder der Implantate können aus gewobenen oder gewirkten Polymerfasern hergestellt sein. Die Abstandselemente können, wie bereits angedeutet, auch in einer textilen Tasche angeordnet sein. Durch eine Tasche wird insbesondere eine Stabilität des Implantats durch gezielte Verstärkung der Abstandselemente erhöht. Eine Faserverstärkung der Abstandselemente kann auch einen Kontaktbereich zwischen den Dornfortsätzen 50 und 52 und den Abstandselementen optimieren, um zum Beispiel einen Abrieb der Abstandselemente an der jeweiligen Kontaktfläche zu minimieren.

Wie oben bereits beschrieben, können die Abstandselemente infolge einer Änderung von Umgebungsbedingungen und/oder auf sie einwirkender Kräfte ihre äußere Gestalt und/oder ihre elastischen Eigenschaften auch anisotrop ändern. Zum Beispiel kann eine Ausdehnung bei der Hydrierung in bestimmten Raumrichtungen behindert werden. Eine anisotrope Struktur der Abstandselemente kann beispielsweise durch einen sandwichartigen Aufbau aus unterschiedlichen Schichten geschaffen werden. Zu diesem Zweck können in die weichere Struktur eines Hydrogels beispielsweise Schichten höherer Steifigkeit eingebracht werden, um eine Gesamtsteifigkeit des Hydrogelkörpers gezielt zu beeinflussen. Hierbei können insbesondere textile Schichten verwendet werden.

Zum Verschließen des Bandes dient, wie oben beschrieben, eine Verschlussvorrichtung. Anstatt des beschriebenen unidirektionalen Verschlusses in Kabelbinderart können auch Pressklemmen, zum Beispiel druckknopfartige Verbindungen vorgesehen sein, oder freie Enden des Bands können einfach vernäht werden.

Zum Einbringen des Implantats wird es, wie oben beschrieben, vormontiert und in die Implantationsstellung gebracht, das heißt die Abstandselemente werden, wenn sie durch Hydrogele gebildet werden, dehydriert. Das supraspinale Ligament des Bewegungssegments bleibt erhalten, das interspinale Ligament zwischen den Dornfortsätzen wird jedoch durchtrennt beziehungsweise stumpf penetriert. Mit einem Messinstrument oder einem Probekörper kann optional ein Abstand zwischen den Dornfortsätzen gemessen und eine entsprechende Implantatgröße ausgewählt werden. Die freien Enden der Bänder werden mit einer Art Nahtinstrument um die Dornfortsätze 50 und 52 geführt. Die Abstandselemente werden von lateral unter das supraspinale Ligament eingeschoben, an den gewünschten Stellen plaziert und eventuell für eine primärstabile Plazierung am interspinalen oder supraspinalen Ligament angenäht. Anschließend wird das Band gespannt, beispielsweise mit einem Spanninstrument zum definierten Vorspannen, und die Verschlussvorrichtung geschlossen, insbesondere durch Verpressen, Clipsen, Vernähen oder Schließen eines Kabelbinderverschlusses.

Nach dem Implantieren hydrieren die Abstandselemente aus Hydrogel durch den osmotischen Druck im extrazellulären Raum. Die Wasseraufnahme kann dabei mehrere Stunden dauern und das Volumen kann um circa das 3- bis 20-fache zunehmen. Durch die Expansion passen sich die Abstandselemente ideal an die anatomischen Strukturen der Dornfortsätze 50 und 52 an. Eine Spannung des Bandes erhöht sich durch Expansion der äußeren kleinen Abstandselemente. Zur Sicherung des Implantats bis zur vollständigen Hydrierung beziehungsweise bis zur Herstellung eines Gleichgewichtszustands, kann es mit vorzugweise resorbierbaren Fäden am Bewegungssegment fixiert werden.

Die oben beschriebenen Implantate weisen insbesondere die Eigenschaft auf, dass sie sich an eine zyklische Hydrierung und Dehydrierung der Bandscheide in der beschriebenen Weise anpassen.

## Patentansprüche

1. Implantat (10; 70; 110) zur dorsalen Stabilisierung einer menschlichen oder tierischen Wirbelsäule (12) umfassend eine Befestigungsvorrichtung (14; 114) zum Anlegen und/oder Festlegen an Dornfortsätzen (50, 52) benachbarter Wirbel (54, 56) der Wirbelsäule (12), welches Implantat (10; 70; 110) mindestens ein der Befestigungsvorrichtung (14; 114) zugeordnetes Abstandselement (34, 36, 38; 134, 136, 138) umfasst, welches derart ausgebildet ist, dass es infolge einer Änderung von Umgebungsbedingungen und/oder auf es einwirkender Kräfte seine äußere Gestalt und/oder seine elastischen Eigenschaften ändert, **dadurch gekennzeichnet, dass** das Abstandselement (34, 36, 38; 134, 136, 138) hydrierbar ist, dass das mindestens eine hydrierbare Abstandselement (34, 36, 38; 134, 136, 138) mindestens teilweise aus einem Hydrogel hergestellt ist, dass das mindestens eine Abstandselement (34, 36, 38; 134, 136, 138) anisotrop verformbar ist, so dass es seine Form und/oder ein umschlossenes Volumen infolge einer Änderung mindestens einer Umgebungsbedingung anisotrop ändert, und dass das mindestens eine anisotrop verformbare Abstandselement (34, 36, 38; 134, 136, 138) seine Form und/oder ein umschlossenes Volumen infolge einer Hydrierung anisotrop ändert.

2. Implantat nach Anspruch 1, **dadurch gekennzeichnet, dass** das mindestens eine Abstandselement (34, 36, 38; 134, 136, 138) in einer Implantationsstellung des Implantats (10; 70; 110), in weicher es in einen menschlichen oder tierischen Körper einführbar ist, dehydriert ist.

3. Implantat nach einem der voranstehenden Ansprüche, **dadurch gekennzeichnet, dass** das mindestens eine Abstandselement (34, 36, 38; 134, 136, 138) derart ausgebildet ist, dass es aufgrund osmotischen Drucks im extrazellulären Raum eines menschlichen oder tierischen Körpers hydrierbar ist.

4. Implantat nach einem der voranstehenden Ansprüche, **dadurch gekennzeichnet, dass** das mindestens eine Abstandselement (34, 36, 38; 134, 136, 138) derart ausgebildet ist, dass es infolge einer Druckeinwirkung mindestens teilweise dehydrierbar ist.

5. Implantat nach einem der voranstehenden Ansprüche, **dadurch gekennzeichnet, dass** das Implantat (10; 70; 110) von einer Implantationsstellung, in welcher es an mindestens einen Dornfortsatz (50, 52) eines Wirbels (54, 56) der Wirbelsäule (12) anlegbar und/oder von diesem lösbar ist, in eine Stabilisierungsstellung bringbar ist, in welcher es an dem mindestens einen Dornfortsatz (50, 52) festlegbar ist.

6. Implantat nach Anspruch 5, **dadurch gekennzeichnet, dass** das Implantat (10; 70; 110) mindestens eine Dornfortsatzaufnahme (18, 20; 118; 120) aufweist, in und/oder an welcher in der Stabilisierungsstellung mindestens einer von zwei Dornfortsätzen (50, 52) zweier benachbarter Wirbel (54, 56) gehalten ist.

7. Implantat nach Anspruch 6, **dadurch gekennzeichnet, dass** mindestens zwei Abstandselemente (34, 36, 38; 134, 136, 138) vorgesehen sind, die an einer Dornfortsatzaufnahme (18, 20; 118, 120) einander diametral gegenüberliegend angeordnet sind.

8. Implantat nach einem der voranstehenden Ansprüche, **dadurch gekennzeichnet, dass** das mindestens eine Abstandselement (34, 36, 38; 134, 136, 138) in einer Grundzustellung ein Volumen aufweist, welches mindestens 3mal kleiner ist als ein Volumen des Abstandselements (34, 36, 38; 134, 136, 138) in einer maximalen Expansionsstellung.

9. Implantat nach einem der voranstehenden Ansprüche, **dadurch gekennzeichnet, dass** das mindestens eine Abstandselement (134) mindestens zwei Abstandselementteile (134a, 134b) umfasst.

10. Implantat nach Anspruch 9, **dadurch gekennzeichnet, dass** die mindestens zwei Abstandselementteile (134a, 134b) unterschiedlich anisotrop hydrierbar sind, so dass sie ihre Form und/oder ein umschlossenes Volumen infolge einer Hydrierung anisotrop ändern.

11. Implantat nach Anspruch 10, **dadurch gekennzeichnet, dass** die mindestens zwei Abstandselementteile (134a, 134b) derart geformt und miteinander verbunden sind, dass sie infolge einer Hydrierung eine Form und/oder ein umschlossenes Volumen in zwei voneinander linear unabhängigen Richtungen ändern.

12. Implantat nach einem der Ansprüche 9 bis 11, **dadurch gekennzeichnet, dass** die mindestens zwei Abstandselementteile (134a, 134b) schichtförmig übereinander angeordnet sind.

13. Implantat nach einem der voranstehenden Ansprüche, **dadurch gekennzeichnet, dass** das mindestens eine Abstandselement (34, 36, 38; 134, 136, 138) eine Abstandselementhülle und einen Abstandselementkern umfasst und dass der Abstandselementkern infolge einer Änderung von Umgebungsbedingungen und/oder auf ihn einwirkender Kräfte seine äußere Gestalt und/oder seine elastischen Eigenschaften ändert.

14. Implantat nach einem der Ansprüche 5 bis 13, **dadurch gekennzeichnet, dass** die Befestigungsvorrichtung (14; 114) mindestens eine Verschlussvorrichtung (22; 122) aufweist und dass mit der mindestens einen Verschlussvorrichtung (22; 122) die mindestens eine Dornfortsatzaufnahme (18, 20; 118, 120) in der Implantationsstellung öffenbar und in der Stabilisierungsstellung ringförmig verschließbar ist.

15. Implantatsystem zur dorsalen Stabilisierung einer menschlichen oder tierischen Wirbelsäule (12) mit mindestens einem Implantat (10; 70; 110) zur dorsalen Stabilisierung der Wirbelsäule (12) umfassend eine Befestigungsvorrichtung (14; 114) zum Anlegen und/oder Festlegen an Dornfortsätzen (50, 52) benachbarter Wirbel (54, 56) der Wirbelsäule (12) und mit einem Instrumentarium zum Einsetzen des Implantats (10; 70; 110) in einen menschlichen oder tierischen Körper, **dadurch gekennzeichnet, dass** das Implantat (10; 70; 110) ein Implantat (10; 70; 110) nach einem der voranstehenden Ansprüche ist.

## Claims

1. Implant (10; 70; 110) for the dorsal stabilization of a human or animal spinal column (12), comprising an attachment device (14; 114) for at least one of placing in position against and fixing to spinous processes (50, 52) of adjacent vertebrae (54, 56) of the spinal column (12), which implant (10; 70; 110) comprises at least one spacer element (34, 36, 38; 134, 136, 138) associated with the attachment device (14; 114), which is designed in such a manner that it alters its external shape and/or its elastic properties as a result of a change in ambient conditions and/or forces acting on it, **characterized in that** the spacer element (34, 36, 38; 134, 136, 138) is adapted to be hydrated, **in that** the at least one hydratable spacer element (34, 36, 38; 134, 136, 138) is produced at least partially from a hydrogel, **in that** the at least one spacer element (34, 36, 38; 134, 136, 138) is deformable anisotropically so that it alters its shape and/or an enclosed volume anisotropically as a result of a change in at least one ambient condition, and **in that** the at least one anisotropically deformable spacer element (34, 36, 38; 134, 136, 138) alters its shape and/or an enclosed volume anisotropically as a result of hydration.

2. Implant as defined in claim 1, **characterized in that** the at least one spacer element (34, 36, 38; 134, 136, 138) is dehydrated in an implantation position of the implant (10; 70; 110), said implant being insertable into a human or animal body in said implantation position.

3. Implant as defined in any one of the preceding claims, **characterized in that** the at least one spacer element (34, 36, 38; 134, 136, 138) is designed in such a manner that it is adapted to be hydrated in the extracellular space of a human or animal body on account of osmotic pressure.

4. Implant as defined in any one of the preceding claims, **characterized in that** the at least one spacer element (34, 36, 38; 134, 136, 138) is designed in such a manner that it is adapted to be dehydrated at least partially as a result of pressure acting on it.

5. Implant as defined in any one of the preceding claims, **characterized in that** the implant (10; 70; 110) is adapted to be brought from an implantation position, said implant being adapted to be placed in position against at least one spinous process (50, 52) of a vertebra (54, 56) of the spinal column (12) and/or be released from it in said implantation position, into a stabilizing position, said implant being adapted to be fixed on the at least one spinous process (50, 52) in said stabilizing position.

6. Implant as defined in claim 5, **characterized in that** the implant (10; 70; 110) has at least one spinous process receptacle (18, 20; 118; 120), at least one of two spinous processes (50, 52) of two adjacent vertebrae (54, 56) being held in and/or at said receptacle in the stabilizing position.

7. Implant as defined in claim 6, **characterized in that** at least two spacer elements (34, 36, 38; 134, 136, 138) are provided, said at least two spacer elements being arranged at a spinous process receptacle (18, 20; 118; 120) so as to be located diametrically opposite one another.

8. Implant as defined in any one of the preceding claims, **characterized in that** the at least one spacer element (34, 36, 38; 134, 136, 138) has in a basic position a volume at least 3 times smaller than a volume of the spacer element (34, 36, 38; 134, 136, 138) in a maximum expansion position.

9. Implant as defined in any one of the preceding claims, **characterized in that** the at least one spacer element (134) comprises at least two spacer element parts (134a, 134b).

10. Implant as defined in claim 9, **characterized in that** the at least two spacer element parts (134a, 134b) are adapted to be anisotropically hydrated differently so that they alter their shape and/or an enclosed volume anisotropically as a result of hydration.

11. Implant as defined in claim 10, **characterized in that** the at least two spacer element parts (134a, 134b) are shaped and connected to one another in such a manner that they alter a shape and/or an enclosed volume in two directions linearly independent of one another as a result of hydration.

12. Implant as defined in any one of claims 9 to 11, **characterized in that** the at least two spacer element parts (134a, 134b) are arranged in layers one on top of the other.

13. Implant as defined in any one of the preceding claims, **characterized in that** the at least one spacer element (34, 36, 38; 134, 136, 138) comprises a spacer element sleeve and a spacer element core and **in that** the spacer element core alters at least one of its external shape and its elastic properties as a result of a change in ambient conditions and/or forces acting on it.

14. Implant as defined in any one of claims 5 to 13, **characterized in that** the attachment device (14; 114) has at least one closure device (22; 122) and **in that** the at least one spinous process receptacle (18, 20; 118; 120) is adapted to be opened in the implantation position and closed in a ring shape in the stabilizing position with the at least one closure device (22; 122).

15. Implant system for the dorsal stabilization of a human or animal spinal column (12) with at least one implant (10; 70; 110) for the dorsal stabilization of the spinal column (12), comprising an attachment device (14; 114) for placing in position against and/or fixing to spinous processes (50, 52) of adjacent vertebrae (54, 56) of the spinal column (12) and with instrumentation for inserting the implant (10; 70; 110) into a human or animal body, **characterized in that** the implant (10; 70; 110) is an implant (10; 70; 110) as defined in any one of the preceding claims.

## Revendications

1. Implant (10; 70; 110) destiné à assurer une stabilisation dorsale d'une colonne vertébrale humaine ou animale (12), comprenant un dispositif de fixation (14; 114) destiné à être appliqué et/ou fixé sur des apophyses épineuses (50, 52) de vertèbres mutuellement voisines (54, 56) de la colonne vertébrale (12), ledit implant (10; 70; 110) comprenant au moins un élément d'espacement (34, 36, 38; 134, 136, 138) associé au dispositif de fixation (14; 114) et conçu de façon à modifier sa configuration extérieure et/ou ses propriétés d'élasticité à la suite d'une variation des conditions environnantes et/ou des forces agissant sur lui, **caractérisé en ce que** l'élément d'espacement (34, 36, 38; 134, 136, 138) est hydratable, **en ce que** ledit au moins un élément d'espacement (34, 36, 38; 134, 136, 138) hydratable est fabriqué au moins partiellement en un hydrogel, **en ce que** ledit au moins un élément d'espacement (34, 36, 38; 134, 136, 138) est déformable de manière anisotrope, de sorte que qu'il modifie de manière anisotrope sa forme et/ou un volume enfermé suite à une variation d'au moins une condition environnante, et **en ce que** ledit au moins un élément d'espacement (34, 36, 38; 134, 136, 138) déformable de manière anisotrope, modifie de manière anisotrope sa forme et/ou un volume enfermé, suite à une hydratation.

2. Implant selon la revendication 1, **caractérisé en ce que** ledit au moins un élément d'espacement (34, 36, 38; 134, 136, 138) est déshydraté pour une position d'implantation de l'implant (10; 70; 110), dans laquelle il peut être introduit dans un corps humain ou animal.

3. Implant selon l'une des revendications précédentes, **caractérisé en ce que** ledit au moins un élément d'espacement (34, 36, 38; 134, 136, 138) est conçu de façon à être hydratable en raison d'une pression osmotique dans le milieu extracellulaire d'un corps humain ou animal.

4. Implant selon l'une des revendications précédentes, **caractérisé en ce que** ledit au moins un élément d'espacement (34, 36, 38; 134, 136, 138) est conçu de façon à pouvoir être au moins partiellement déshydraté suite à l'action d'une compression.

5. Implant selon l'une des revendications précédentes, **caractérisé en ce que** l'implant (10; 70; 110) peut être amené d'une position d'implantation, dans laquelle il peut être appliqué sur au moins une apophyse épineuse (50, 52) d'une vertèbre (54, 56) de la colonne vertébrale (12) et/ou en être détaché, dans une position de stabilisation, dans laquelle il peut être fixé à ladite au moins une apophyse épineuse (50, 52).

6. Implant selon la revendication 5, **caractérisé en ce que** l'implant (10; 70; 110) comprend au moins un logement d'accueil d'apophyse épineuse (18, 20; 118, 120) dans et/ou sur lequel est maintenue, dans la position de stabilisation, au moins l'une de deux apophyses épineuses (50, 52) de deux vertèbres mutuellement voisines (54, 56).

7. Implant selon la revendication 6, **caractérisé en ce que** sont prévus au moins deux éléments d'espacement (34, 36, 38; 134, 136, 138), qui sont agencés de manière diamétralement opposée sur un logement d'accueil d'apophyse épineuse (18, 20; 118, 120).

8. Implant selon l'une des revendications précédentes, **caractérisé en ce que** ledit au moins un élément d'espacement (34, 36, 38; 134, 136, 138) présente, dans une position initiale, un volume qui est au moins trois fois moindre qu'un volume de l'élément d'espacement (34, 36, 38; 134, 136, 138) dans une position d'expansion maximale.

9. Implant selon l'une des revendications précédentes, **caractérisé en ce que** ledit au moins un élément d'espacement (134) comprend au moins deux parties d'élément d'espacement (134a, 134b).

10. Implant selon la revendication 9, **caractérisé en ce que** lesdites au moins deux parties d'élément d'espacement (134a, 134b) sont hydratables de manière anisotrope différente, de sorte qu'elles modifient de manière anisotrope leur forme et/ou un volume enfermé, suite à une hydratation.

11. Implant selon la revendication 10, **caractérisé en ce que** lesdites au moins deux parties d'élément d'espacement (134a, 134b) sont formées et reliées mutuellement de manière telle, que suite à une hydratation, elles modifient une forme et/ou un volume enfermé dans deux directions linéairement indépendantes l'une de l'autre.

12. Implant selon l'une des revendications 9 à 11, **caractérisé en ce que** lesdites au moins deux parties d'élément d'espacement (134a, 134b) sont agencées de manière superposée sous forme de couches.

13. Implant selon l'une des revendications précédentes, **caractérisé en ce que** ledit au moins un élément d'espacement (34, 36, 38; 134, 136, 138) comprend une enveloppe d'élément d'espacement et une âme d'élément d'espacement, et **en ce que** l'âme d'élément d'espacement modifie sa configuration extérieure et/ou ses propriétés d'élasticité à la suite d'une variation de conditions environnantes et/ou de forces agissant sur elle.

14. Implant selon l'une des revendications 5 à 13, **caractérisé en ce que** le dispositif de fixation (14; 114) comporte au moins un dispositif de fermeture (22; 122), et **en ce qu'**à l'aide dudit au moins un dispositif de fermeture (22; 122) ledit au moins un logement d'accueil d'apophyse épineuse (18, 20; 118, 120) peut être ouvert dans la position d'implantation, et être fermé en forme de boucle, dans la position de stabilisation.

15. Système d'implant pour la stabilisation dorsale d'une colonne vertébrale humaine ou animale (12), comprenant au moins un implant (10; 70; 110) pour la stabilisation dorsale de la colonne vertébrale (12), qui comporte un dispositif de fixation (14; 114) destiné à être appliqué et/ou fixé sur des apophyses épineuses (50, 52) de vertèbres mutuellement voisines (54, 56) de la colonne vertébrale (12), et comprenant un ensemble d'instruments pour la mise en place de l'implant (10; 70; 110) dans un corps humain ou animal, **caractérisé en ce que** l'implant (10; 70; 110) est un implant (10; 70; 110) selon l'une des revendications précédentes.
